Europäisches Patentamt

European Patent Office

Office européen des brevets

(11) Numéro de publication: **0 284 530**
**A1**

## (12) DEMANDE DE BREVET EUROPEEN

(21) Numéro de dépôt: **88440014.4**

(22) Date de dépôt: **04.03.88**

(51) Int. Cl.4: **A 61 F 5/02**

(30) Priorité: **27.03.87 FR 8704456**

(43) Date de publication de la demande:
**28.09.88 Bulletin 88/39**

(84) Etats contractants désignés:
**AT BE CH DE ES GB GR IT LI LU NL SE**

(71) Demandeur: **Razafindratsiva, César**
**15 Avenue du Ray**
**F-06000 Nice (FR)**

(72) Inventeur: **Razafindratsiva, César**
**15 Avenue Du Ray**
**F-06100 Nice (FR)**

**Bohatier, Claude**
**18 Avenue des Bosquets**
**F-06000 Nice (FR)**

**Mouraret, Michel**
**113 blvd de la Plage**
**F-06800 Cagnes Sur Mer (FR)**

**Le Floch, Alain**
**19 Rue Henri Bosco**
**F-06560 Valbonne (FR)**

**Tomasoni, Lucien**
**29 Corniche Frère Marc**
**F-06000 Nice (FR)**

**D'Hondt, Didier**
**42 Blvd Napoléon III**
**F-06200 Nice (FR)**

(74) Mandataire: **Hautier, Jean-Louis**
**Cabinet Hautier 24 rue Masséna**
**F-06000 Nice (FR)**

(54) **Plaque de rachis.**

(57) Plaque de rachis, destinée à permettre l'ostéosynthèse des vertèbres des zones inférieures de la colonne vertébrale après une intervention chirurgicale intéressant l'une de ces zones, caractérisée en ce qu'elle se compose de deux lames sensiblement verticales réunies à leurs extrémités par des ponts transversaux, chacune de ces lames comportant des fenêtres longitudinales dont la forme et les dimensions sont prévues de manière à permettre le passage de vis de fixation, chacune au niveau optimal pour chaque patient.

1/3

FIG. 1

**Description**

PLAQUE DE RACHIS.

La présente invention concerne au titre de produit industriel nouveau, une plaque destinée à permettre dans les meilleures conditions l'ostéosynthèse du rachis, c'est-à-dire la reconstruction des zones inférieures de la colonne vertébrale.

On sait que dans les interventions chirurgicales consécutives à des accidents ayant affecté les zones inférieures de la colonne vertébrale, il est usuel d'immobiliser les vertèbres intéressées pour permettre la reconstitution progressive des portions lésées. A cet effet, on utilise actuellement des barrettes métalliques perforées à intervalles réguliers de l'ordre de 10 à 15 mm, et fixées sur les pédicules des vertèbres au moyen de vis traversant ces perforations.

Ce système présente divers inconvénients.

En premier lieu, il n'offre guère de souplesse de réglage, en dépit des différences morphologiques entre les individus et les zones affectées. En effet, l'espacement des vis est fixe et relativement considérable, ce qui impose des contraintes gênantes dans la direction verticale.

D'autre part, dès lors qu'il s'agit de barrettes linéaires sensiblement verticales, ce système ne saurait assurer une rigidification complète de la zone osseuse intéressée puisque l'immobilisation de cette zone est limitée à une seule direction.

Enfin, les barrettes actuelles sont relativement épaisses et lourdes, et trop rigides, ce qui représente un inconfort certain pour le patient. L'invention remédie à tous ces inconvénients, et présente au surplus un certain nombre de caractéristiques avantageuses supplémentaires. A cet effet, l'invention vise une plaque dite "plaque de rachis", caractérisée en ce qu'elle se compose de deux lames sensiblement verticales réunies par des ponts traversaux, chacune de ces lames comportant des fenêtres longitudinales ajourées pour le passage de vis de fixation aux niveaux choisis par l'opérateur, et chaque pont transversal comportant de préférence des moyens de réglage en largeur de l'écartement entre ces lames également en fonction des besoins puis de rigidification à la valeur définitive à la fin de l'intervention chirurgicale.

Selon des caractéristiques secondaires :

- chaque fenêtre longitudinale présente la forme d'une succession de cercles jointifs ayant chacun le diamètre d'une vis, et d'un évasement ayant le diamètre de la tête d'une vis, de manière à permettre de positionner, dans chaque fenêtre, une vis à tout niveau convenable selon l'intervention indifféremment sur toute la longueur de ladite fenêtre.

- chaque pont transversal se compose d'une tige transversale, solidaire d'une lame à distance correspondant à l'épaisseur de la vertèbre, et venant s'insérer dans un logement lui faisant face dans l'autre lame, le coulissement de cette tige dans ce logement permettant de modifier la largeur du pont, et pouvant être bloqué par tout moyen mécanique convenable quand ladite largeur a été amenée à la valeur nécessaire dans chaque cas.

- les couples de lames sont galbées en fonction de l'emplacement sur lequel elles doivent être adaptées, et notamment, de haut en bas, la zone dorso-lombaire, la zone lombaire et la zone lombo-sacrée.

- enfin la plaque de rachis selon l'invention est réalisée en acier au chrome ou en titane.

Grâce à ces diverses caractéristiques, le chirurgien peut, quelle que soit la zone lésée et la morphologie propre du patient, adapter un système unique aux conditions particulières à l'intervention qu'il doit pratiquer, et "rigidifier" ce système après ladite adaptation, et une fois ladite intervention achevée. Le système selon l'invention est en outre beaucoup plus léger et confortable que les systèmes antérieurs connus.

On va maintenant illustrer l'invention en se référant au dessin annexé sur lequel :

- la figure 1 est une vue de face d'une plaque de rachis selon l'invention, montrée sans moyen de réglage transversal,

- la figure 2 est une vue latérale de la plaque de la figure 1, dans le cas d'une plaque dorso-lombaire,

- la figure 3 est une coupe suivant III-III de la figure 1 et

- la figure 4 illustre un moyen de réglage d'un pont transversal adaptable sur la plaque précédente.

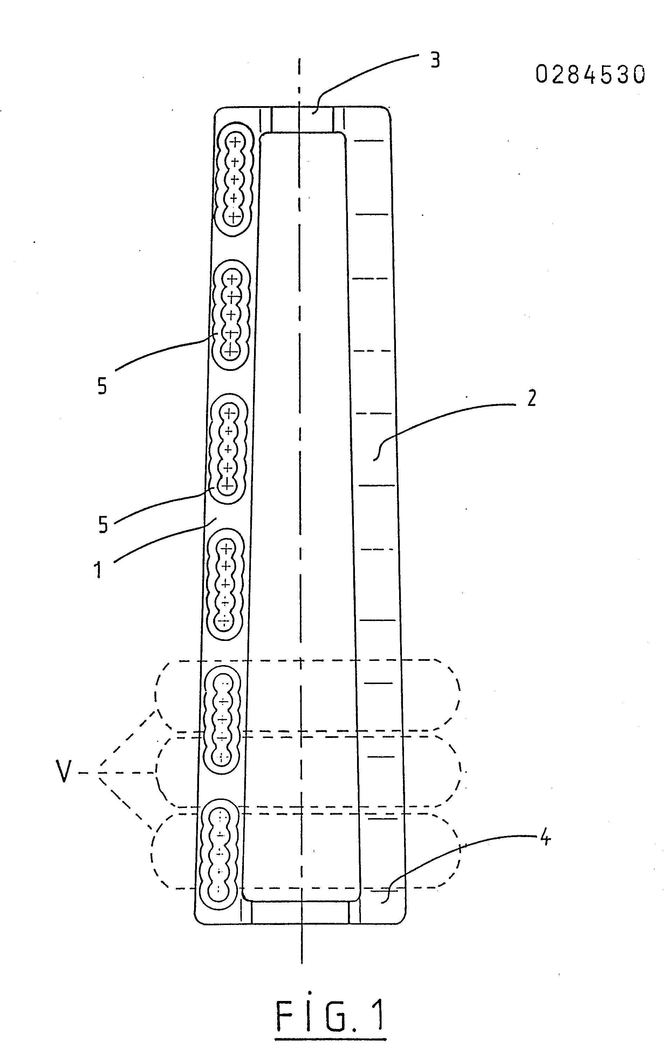

Si l'on se réfère d'abord aux figures 1 à 3, on voit que la plaque de rachis selon l'invention se compose de deux lames sensiblement verticales 1 et 2, réunies par des ponts transversaux 3 et 4, représentés ici chacun en une seule pièce solidaire des lames 1 et 2, c'est-à-dire non réglable en dimension.

Dans le cas illustré ici, d'une plaque dorso-lombaire, le pont 4 est plus large que le pont 3 pour tenir compte des dimensions des vertèbres (représentées ici schématiquement en V).

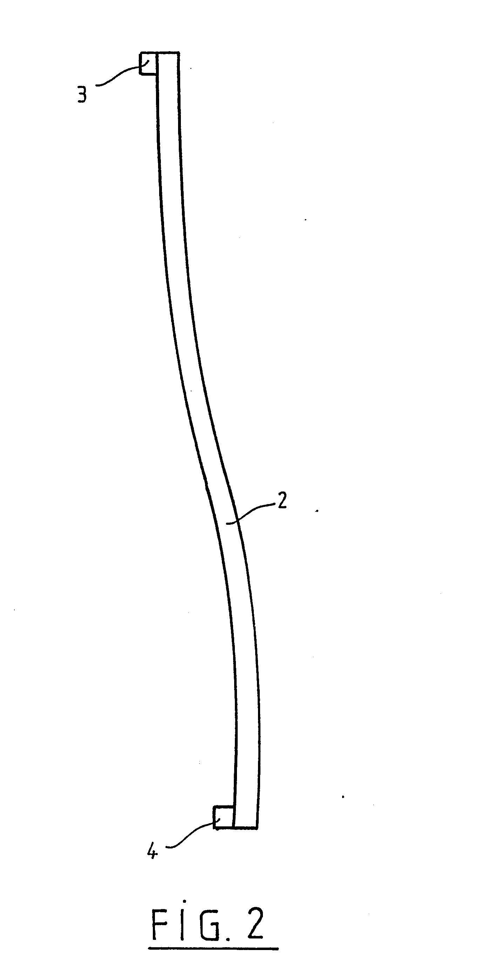

De même, dans ce cas, les lames 1 et 2 sont galbées comme il apparaît sur la figure 2 pour tenir compte du galbe correspondant de la colonne vertébrale dans cette région.

Comme il est représenté en 5 sur la lame 1, des lumières sont pratiquées dans chacune des lames 1 et 2, chacune de ces lumières ayant la forme résultant de la juxtaposition avec un faible chevauchement du diamètre de plusieurs trous sécants (ici 5), les vis s'y insérant devant assurer la fixation de la plaque sur les pédicules des vertèbres V intéressés par cette plaque.

Un premier avantage de l'invention est de permettre le positionnement vertical optimal de chaque vis sur le pédicule correspondant, grâce à cette lumière, au lieu du trou cylindrique unique des barrettes antérieures.

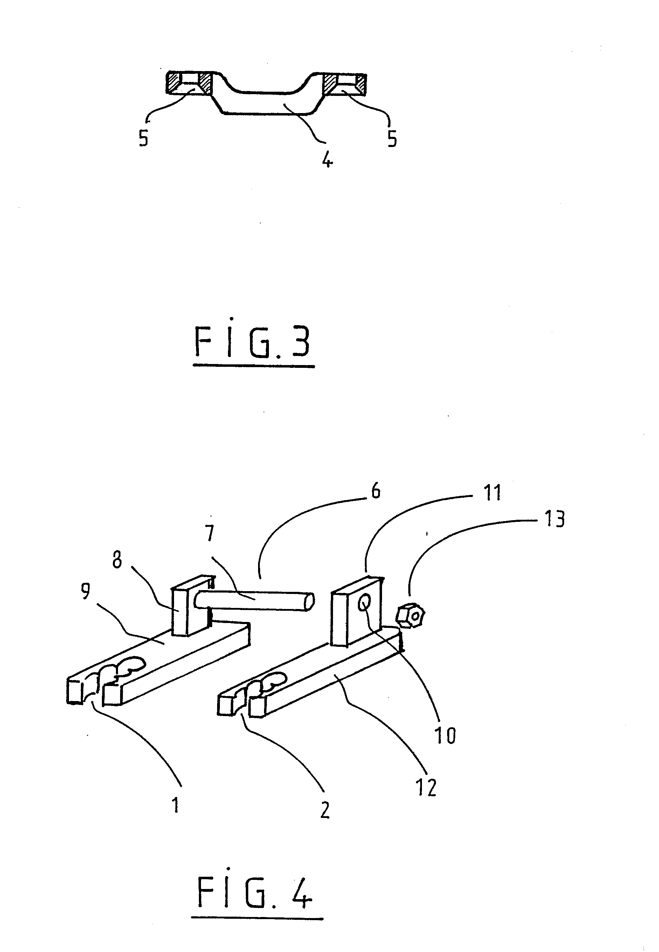

Dans la pratique, par ailleurs, pour assurer un positionnement horizontal optimal, chaque pont 3 ou 4 comporte de préférence, des moyens de réglage en largeur, tels ceux illustrés à la figure 4. Sur la figure 4, le pont 6 se compose d'une tige horizontale 7, solidaire d'une patte 8 perpendiculaire à l'extrémité 9 de la première lame, et venant traverser un logement 10 d'une patte 11, perpendiculaire à l'extrémité 12 de la seconde lame.

Un écrou 13 vient se visser sur l'extrémité de la tige 7, de manière à bloquer l'ensemble, après intervention, pour assurer à la plaque des dimensions optimales.

En d'autres termes, une plaque de rachis selon l'invention, après intervention, est entièrement adaptée à la configuration et aux dimensions d'ensemble de la zone traitée, ce réglage étant obtenu par déformation par exemple au moyen d'une pince, puis rigidifiée dans sa configuration.

L'intérêt de l'invention réside donc dans cette possibilité d'adaptation optimale, à partir d'un matériel standardisé, donc moins coûteux par rapport aux résultats atteints. Ainsi, par exemple, tous les cas normalement concevables pourront être couverts de manière personnalisée à partir de trois plaques standard correspondant aux zones dorso-lombaire, lombaire et lombo-sacrée du rachis.

Ces plaques pourront être réalisée en tout matériau approprié, et notamment en acier au chrome ou en titane.

Bien entendu la description qui précède n'est donnée qu'à titre d'exemple illustratif, et de nombreuses variantes pourront en être conçues, restant dans le cadre de l'invention. En particulier des moyens de réglage de la largeur des ponts 3 et 4 autres que celui illustré à la figure 4 pourront être utilisés. De même les lumières 5 pourront avoir des longueurs différentes de cinq fois le diamètre d'une vis, soit d'une plaque à l'autre, soit même sur une même lame, notamment en fonction de la hauteur des pédicules.

## Revendications

1. Plaque de rachis, destinée à permettre l'ostéosynthèse des vertèbres des zones inférieures de la colonne vertébrale après une intervention chirurgicale intéressant l'une de ces zones, caractérisée en ce qu'elle se compose de deux lames sensiblement verticales réunies à leurs extrémités par des ponts transversaux, chacune de ces lames comportant des fenêtres longitudinales dont la forme et les dimensions sont prévues de manière à permettre le passage de vis de fixation, chacune au niveau optimal pour chaque patient.

2. Plaque de rachis selon la revendication 1, caractérisée en ce qu'au moins l'un des ponts transversaux comporte des moyens de réglage de l'écartement entre les lames, et de blocage de ces moyens après l'intervention chirurgicale sans l'intervention d'éléments d'assemblages supplémentaires.

3. Plaque selon les revendications 1 et 2, caractérisée en ce que chaque fenêtre présente la forme d'une succession continue de cercles juxtaposés ayant chacun le diamètre d'une vis de fixation.

4. Plaque selon l'une quelconque des revendications 1 à 3, caractérisée en ce que les moyens de réglage de la longueur d'un pont transversal consistent en une tige horizontale dont les deux extrémités doivent être solidarisées à la fin de l'intervention, l'une d'elle pouvant l'être dès le début de la pose.

5. Plaque selon l'une quelconque des revendications 1 à 4, caractérisée en ce quele couple de lames d'une plaque est galbé conformément à la configuration du patient dans la zone lésée.

6. Plaque selon l'une quelconque des revendications 1 à 4, pouvant être personalisée à la morphologie du patient après analyse par des moyens d'imagerie médicale.

0284530

FIG. 1

0284530

FIG. 2

0284530

FIG.3

FIG.4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | GB-A- 780 652 (ZIMMER ORTHOPAEDIC) <br> * Figure * | 1,2 | A 61 F 5/02 |
| Y |  | 3-6 | |
| X | FR-A-2 099 960 (NATIONAL RESEARCH DEVELOPMENT CORP.) <br> * En entier * | 1 | |
| A |  | 3 | |
| Y | US-A-4 611 581 (A.D. STEFFEE) <br> * Figures 1,9 * | 3 | |
| Y | FR-A-2 516 788 (L.L. RODNYANSKY et al.) <br> * Figure 1 * | 4 | |
| Y | DE-A-2 920 223 (MASCHINENFABRIK - AUGSBURG-NÜRNBERG) <br> * Revendications 1,5,8 * | 5,6 | |
| A | FR-A-2 320 078 (R. LOUIS) <br> * Page 2, lignes 1-8; figures * | 1,3 | DOMAINES TECHNIQUES RECHERCHES (Int. Cl.4) |
| A | DE-A-3 114 136 (AESCULAP-WERKE) <br> * Résumé * | 1 | A 61 F <br> A 61 B |
| A | US-A-4 003 376 (D.W. McKAY et al.) <br> * Résumé * | 5 | |

Le présent rapport a été établi pour toutes les revendications

| Lieu de la recherche | Date d'achèvement de la recherche | Examinateur |
|---|---|---|
| LA HAYE | 09-06-1988 | WOLF C.H.S. |